(19) Europäisches Patentamt
European Patent Office
Office européen des brevets

(11) **EP 1 395 282 B1**

## (12) FASCICULE DE BREVET EUROPEEN

(45) Date de publication et mention
de la délivrance du brevet:
**29.03.2006 Bulletin 2006/13**

(21) Numéro de dépôt: **02738290.2**

(22) Date de dépôt: **30.05.2002**

(51) Int Cl.:
***A61K 39/008*** (2006.01)   ***A61P 33/02*** (2006.01)

(86) Numéro de dépôt international:
**PCT/FR2002/001823**

(87) Numéro de publication internationale:
**WO 2002/100430 (19.12.2002 Gazette 2002/51)**

(54) **COMPLEXE VACCINAL DESTINE A LA PREVENTION ET AU TRAITEMENT DES LEISHAMANIOSES**

IMPFKOMPLEX ZUR VORBEUGUNG UND BEHANDLUNG VON LEISHMANIASIS

VACCINE COMPLEX FOR PREVENTING OR TREATING LEISHMANIASES

(84) Etats contractants désignés:
**AT BE CH CY DE DK ES FI FR GB GR IE IT LI LU MC NL PT SE TR**

(30) Priorité: **11.06.2001 FR 0107606**

(43) Date de publication de la demande:
**10.03.2004 Bulletin 2004/11**

(73) Titulaire: **Bio Veto Tests, en abrégé BVT (SARL) 83500 La Seyne sur Mer (FR)**

(72) Inventeurs:
• **PAPIEROK, Gérard**
  **F-83400 Hyères (FR)**
• **VICENS, Serge**
  **F-13180 Gignac la Nerthe (FR)**
• **LEMESRE, Jean-Loup**
  **F-34000 Montpellier (FR)**

(74) Mandataire: **Marek, Pierre et al Cabinet Marek, 28 & 32, rue de la Loge 13002 Marseille (FR)**

(56) Documents cités:
**WO-A-94/26899**

• **CIBRELUS P ET AL: "Secreted antigens of the amastigote and promastigote forms of Leishmania infantum inducing a humoral response in humans and dogs." PARASITE, vol. 6, no. 2, juin 1999 (1999-06), pages 121-129, XP008007414 ISSN: 1252-607X**

• **MERLEN T ET AL: "Leishmania spp: completely defined medium without serum and macromolecules (CDM/LP) for the continuous in vitro cultivation of infective promastigote forms." THE AMERICAN JOURNAL OF TROPICAL MEDICINE AND HYGIENE, vol. 60, no. 1, janvier 1999 (1999-01), pages 41-50, XP008007415 ISSN: 0002-9637**

• **FROMMEL D ET AL: "Vaccine-induced immunity against cutaneous leishmaniasis in BALB/c mice" INFECTION AND IMMUNITY, vol. 56, no. 4, 1988, pages 843-848, XP002023859 ISSN: 0019-9567**

• **STEINBERGER A ET AL: "Leishmania tropica protective response in C3H mice vaccinated with excreted factor crosslinked with the synthetic adjuvant muramyl dipeptide" EXPERIMENTAL PARASITOLOGY, vol. 58, no. 3, 1984, pages 223-229, XP008007419 ISSN: 0014-4894**

• **GUILHERME PRESTES BEYRODT C ET AL: "Characterization of an antigen from Leishmania amazonensis amastigotes able to elicit protective responses in a murine model." INFECTION AND IMMUNITY, vol. 65, no. 6, 1997, pages 2052-2059, XP002118939 ISSN: 0019-9567**

Remarques:
Le dossier contient des informations techniques présentées postérieurement au dépôt de la demande et ne figurant pas dans le présent fascicule.

**EP 1 395 282 B1**

**Description**

La présente invention concerne un complexe immunomodulateur spécifique composé d'antigènes d'excrétion et sécrétion de Leishmania et son utilisation dans les infections à microorganismes pathogènes intracellulaires chez le mammifère et en particulier chez l'homme, les canidés, les félidés et les équidés.

Plus précisément, la présente invention concerne un complexe vaccinal thérapeutique destiné à la prévention ou au traitement des leishmanioses et des infections à micro-organismes pathogènes intracellulaires chez le mammifère et en particulier chez l'homme, les canidés, les félidés et les équidés, ce complexe vaccinal étant notamment remarquable en ce qu'il est composé de molécules d'excrétion sécrétion issues d'amastigotes et/ou de promastigotes de Leishmania sp. produites dans un milieu axénique et asérique défini.

Les leishmanioses constituent un groupe d'infections parasitaires endémiques voire épidémiques des régions tropicales et subtropicales du monde. Les *leishmania,* protozoaires flagellés de la famille des trypanosomatidae et du genre *Leishmania,* sont les agents pathogènes responsables de ces maladies. Ces parasites affectent de très nombreuses espèces de mammifères dont l'homme et le chien qui constituent les principaux réservoirs domestiques de ces maladies. Les leishmanies sont transmises à ces différents hôtes lors de la piqûre infectante d'un petit moucheron que l'on appelle phlébotome. Dix-neuf espèces de leishmanies sont potentiellement capables d'infecter l'homme et selon les espèces de leishmanies considérées et des facteurs propres à l'hôte (génétique, immunologique... ), elles sont à l'origine de manifestations cliniques très diverses. Elles évoluent principalement vers trois formes cliniques distinctes : Cutanée, Muco-cutanée et Viscérale selon que les parasites affectent le système phagocyte mononuclée du derme, des muqueuses ou des viscères. La lésion cutanée peut rester localisée au point d'inoculation du parasite et correspondre à une forme bénigne à guérison spontanée. A coté de cela il existe des pathologies plus graves provoquées par les leishmanioses cutanéo-diffuses et cutanéo-muqueuses qui sont très mutilantes et défigurantes.

La leishmaniose viscérale affecte le système phagocyte mononuclée de nombreux organes et tissus et notamment le foie, la rate et la moelle osseuse (hépatomégalie et splénomégalie) et est fatale en l'absence de traitement.

Comme toutes les maladies à transmission vectorielle, les leishmanioses se caractérisent par un cycle évolutif qui est relativement simple puisqu'il se partage entre deux hôtes, mammifère et phlébotomes, et comprend deux formes principales :

- une forme flagellée appelée promastigote, présente dans le tube digestif du vecteur phlébotome où il se multiplie avant d'acquérir sa forme infectante pour l'hôte mammifère, encore appelée métacyclique ;

- une forme non flagellée appelée amastigote, présente chez l'hôte mammifère dont le chien et l'homme.

Le phlébotome vit dans les régions chaudes du globe (climat chaud méditerranéen ou tropical). Pour se développer, il exige une température supérieure à 17°C (conditions idéales entre 22 à 25°C) une atmosphère humide et l'absence de vent.

Les zones suburbaines des pays méditerranéens, où la présence de chiens est plus importante, réunissent les conditions environnementales adéquates pour que des phlébotomes puissent se reproduire (tas de fumier, fermes, jardins, abris boisés, murs, pelouses arrosées...), ce qui favorise une plus grande densité d'insectes à proximité des chiens domestiques et de l'homme.

Les leishmanioses représentent encore aujourd'hui un important problème de santé publique en particulier dans les pays en développement, et un remarquable sujet d'étude et de recherche aussi bien fondamentale qu'appliquée en particulier dans le domaine de l'immunoprophylaxie. Quatre vingt dix sept pays répartis sur 4 des 5 continents sont concernés par les leishmanioses. Menaçant quelque 380 millions de personnes à travers le monde, ces parasitoses affectent environ 18 millions de personnes dans le monde, avec environ 2 millions de nouveaux cas par an, 90% de ces cas étant recensés en Inde, au Soudan et au Brésil. Il y a quinze ans, la fréquence annuelle mondiale était estimée à 400 000 cas [300 000 cas de leishmaniose cutanée (LC) et 100 000 cas de leishmaniose viscérale (LV)], avec une incidence générale de 12 millions de cas cliniques, et une population à risque d'environ 350 millions d'individus. Actuellement, la fréquence annuelle globale est estimée entre 1,5 et 2 millions de nouveaux cas par an, dont 1 à 1,5 millions de cas de LC et 500 000 cas de LV.

Si les populations tropicales et subtropicales sont en première ligne face à ces maladies, les risques de contamination canine et humaine dans le bassin méditerranéen sont souvent sous estimés. La leishmaniose viscérale à *Leishmania infantum* qui est largement répandue sur les différents continents de l'ancien monde, est présente sur tout le pourtour du bassin méditerranéen, le sud de la France constituant un des foyers. Si le vecteur comme le parasite présents dans le sud de la France semblent mieux adaptés au chien qu'à l'homme, le nombre de cas humains de leishmaniose, actuellement estimé à une centaine de cas annuels, est en pleine croissance depuis 10 ans et est encore aggravé par le nombre croissant de sujets immudéprimés.

Les leishmanioses sont aussi considérées comme l'une des maladies opportuniste du SIDA. On dénombre environ 1500 cas de coinfection HIV / Leismanie dans le sud de l'Europe ce qui représente 90 % des cas recensés dans le monde et c'est l'Espagne qui est le pays le plus touché avec environ 60 % des cas.

Le chien domestique est le réservoir principal de parasite. La leishmaniose canine qui est une pathologie courante du pourtour méditerranéen se traduit par diverses formes cliniques qui conduisent souvent à la mort de l'animal. La prévalence de la leishmaniose canine peut atteindre 30% de la population canine dans certaines zones périurbaines. Selon Berrahal et coll (Am. J.Trop.Med.Hyg, 1996, 55, 273-277) 85% des chiens sont PCR (Polymerase Chain Reaction) positifs en zone endémique.

Pour le moment, il n'existe pas de moyens immunoprophylactiques efficaces contre ces maladies. La thérapie des leishmanioses fait appel à quelques molécules disponibles: antimoine pentavalent, pentamidine, pyrazolopyrimidines, amphotéricine B, aminosidine. Aujourd'hui, un consensus semble s'imposer pour considérer l'association sels d'antimoine pyrazolopyrimidines comme le traitement de choix de la leishmaniose canine. Néanmoins, les chiens sous traitement restent infectieux, malgré l'apparente guérison clinique de l'animal.

Cela signifie que l'amélioration symptomatique n'est pas corrélée à la diminution significative de la charge parasitaire et qu'il existe un risque épidémiologique même si la guérison clinique persiste. Cette situation est encore compliquée par l'émergence de phénomènes de chimiorésistance.

A l'heure actuelle, bien que les problèmes de chimiorésistance compliquent considérablement le traitement, on est toujours incapable de déterminer la prévalence de celle-ci en zone d'endémie et de la diagnostiquer chez les patients. De même, les bases moléculaires de cette résistance induite chez le stade médicalement important du parasite (i.e. amastigote) ne sont toujours pas connues.

Enfin, les cas de co-infection Sida / leishmaniose posent un sérieux problème de santé publique dans la mesure où les thérapeutiques disponibles sont peu efficaces chez les malades du sida ainsi que chez toute personne immunodéprimée.

Aujourd'hui, aucun vaccin efficace n'est actuellement disponible pour combattre ces maladies et leur contrôle passe nécessairement par la chimiothérapie. Cette dernière est malheureusement mise en péril par des traitements longs, toxiques et onéreux s'accompagnant de nombreux cas de rechute et par l'émergence des phénomènes de chimiorésistance. Il paraît aujourd'hui évident que le traitement de ces maladies parasitaires dans le long terme dépend de la découverte de nouvelles cibles thérapeutiques et/ou vaccinales.

La présente invention propose un complexe vaccinal thérapeutique spécifique de Leishmania agissant sur la réponse immunitaire de l'hôte mammifère infecté.

Les nombreuses études portant sur les réponses immunitaires au cours des leishmanioses murines expérimentales ont conduit à la démonstration du rôle prépondérant de l'immunité à médiation cellulaire et de l'existence d'une dualité de la réponse immunologique. Il existe fondamentalement deux types de réponses à l'encontre des leishmanies : l'une qualifiée de « sensibilité », l'autre de « résistance ». C'est par le biais des lymphokines qu'elles sécrètent que les différentes sous-populations de lymphocytes T (CD4+) limitent ou exacerbent l'infection. Il a ainsi été démontré que la sous population de lymphocytes T auxiliaires de type Th1 (producteur d'interféron gamma et d'interleukine 2) était capable d'éliminer les formes amastigotes intracellulaires par le biais de l'activation des macrophages (Reiner S.L et al., Annu Rev Immunol, 1995, 13, 151-177. Review). Inversement la sous population de lymphocytes T auxiliaires de type Th2 (producteur d'interleukine 4) est responsable de l'exacerbation de la maladie.

Chez l'homme, certains faits sont de nature comparable. Chez le chien (hôte naturel « réservoir » réceptif au cycle évolutif de *L. infantum*), la dualité de la réponse immunologique est vraisemblable. Seule une étude menée par Pinelli et al. (Infect. immun., 62 :229, 1994) sur des animaux expérimentalement et naturellement infectés par *L. infantum,* a permis de montrer que l'asymptomatisme du chien (état clinique fréquemment rencontré) s'accompagnait de l'absence d'une réponse humorale et du développement d'une immunité à médiation cellulaire de type Th1 avec une réaction d'hypersensibilité de type retardée positive et des taux élevés d'interleukine 2 et de cachectine (TNF-$\alpha$) circulant dans les liquides biologiques.

Un bon candidat vaccinal se doit donc de correspondre à un ou plusieurs antigènes parasitaires fortement immunogène (s) capables soit de bloquer la différenciation des lymphocytes Th2 (Gurunathan S et al., J.Exp Med, 1997 Oct 6, 186, 1137-1147) (mode d'intervention comparable aux traitements de « désensibilisation », couramment pratiqués dans les cas d'allergie), soit de favoriser l'émergence de lymphocytes Th1 assurant la mise en place d'une immunité protectrice.

La présente invention concerne un complexe vaccinal thérapeutique et préventif anti Leishmania qui possède un pouvoir vaccinant lié à la présence d'antigènes d'excrétion sécrétion spécifiques contre Leishmania par activation de la voie Th1.

Envisager de vacciner contre les leishmanies reste encore aujourd'hui problématique. Les tentatives sont nombreuses mais les résultats sont faibles et/ou contradictoires. Nous pouvons citer l'utilisation de parasites vivants, de parasites irradiés et de parasites tués entiers (Moreau Y et coil, 1994, Médecine et Armées, 22, 1, 89-93) qui ont donné des niveaux de protection variable chez les souris et chez l'homme.

Dans les années 80, des extraits purifiés d'antigènes parasitaires ont été utilisés chez le chien en induisant une exacerbation de la maladie : fraction LIF2 et vaccin anti idiotypique de l'équipe du Dr Montjour. (CHAUVY, J « Essais d'immunothérapie sur une population canine en zone d'endémie leishmanienne » thèse n°36.1993-OGUNKOLADE B.W et coll. Vet Parasitol, 1988, 28, 33-41). D'autres antigènes comme les antigènes membranaires GP63 et lipophosphoglucane (MOREAU Y et coll, Médecine et Armées, 1994, 22, 1, 89-93) n'ont pas donné de résultat satisfaisant. Actuellement, plusieurs molécules sont à l'essai et sont en attente de résultat final. Citons la protéine de heat shock HSP83 de Leishmania major qui stimule la voie Th1 et la protéine DP72 (JAFFE.C et al, J of Immunol, 1990, 144, 699-706). Cependant, aucun des protocoles actuels d'immunisation ne permet d'obtenir un niveau suffisant de protection ou n'est en tout cas reproductible.

A ce jour, aucun travail n'a été effectué avec des antigènes d'excrétion sécrétion de Leishmania.

La présente invention consiste en un complexe immunomodulateur utilisant ces protéines d'excrétion sécrétion avec un adjuvant induisant soit une immunostimulation du système lymphocytaire T de type Th 1 de façon reproductible, soit une immunomodulation des lymphocytes de type Th 2 vers un type Th 1.

Chez de nombreux parasites tels que Plasmodium, Babesia, Trypanosoma, Toxoplasma ou Shistosoma, il a été démontré que les antigènes d'excrétion sécrétion (AES) jouaient un rôle prépondérant dans l'établissement de la réponse immune de l'hôte. Les AES de leishmanies semblent impliqués dans la pénétration du macrophage par les parasites, dans l'inhibition d'enzymes protéolytiques lysosomales du macrophage et la régulation négatives des molécules du complexe majeur d'histocompatibilité (Alexander et Russel, Adv. Parasitol., 1992, 31, 175-254). D'ailleurs des approches vaccinales menées chez la souris utilisant les AES ont déjà été envisagées avec succès dans différentes parasitoses (Ouaissi et al, Parasitology, 1990, 100, 115-24; James et al., Trans. R. Soc. Trop. Med. Hyg., 1989, 83, 67-72; Précigout et al, Infect. Immun., 1991, 59, 2799-805; Darcy et al., Ann. Biol. Clin., 1989, 47, 451-7 ; Capron et al., Mem. Inst. Oswaldo Cruz., 1995, 90, 235-4).

Mais la difficulté d'obtention et les nombreux contaminants sériques et/ou cellulaires contenus dans les sumageants de culture rendaient difficile leur utilisation en vaccination.

Le milieu de culture décrit dans la Demande de Brevet d'invention WO 94/26 899 permet de résoudre partiellement ces difficultés et de disposer d'une source abondante, propre et peu coûteuse d'AES des principaux stades parasitaires des leishmanies.

Afin d'obtenir un bon rendement de culture des promastigotes et amastigotes de Leishmanies, le milieu du brevet WO 94/26 899 a été modifié comme suit :

- Pour la culture des amastigotes, l'addition des composés soufrés comme la L-cystéine et/ou des produits nutritifs comme l'acide bathocuproïne sulfonique a été supprimée.

Le milieu MA1 de base avec les composés soufrés est tel que :

| Constituants | Quantités pour 800 ml |
|---|---|
| Milieu de base | |
| - Milieu 199 H ® (x10) ( avec sels de Hanks )* | 100 ml |
| - Trypto-caséine de soja ® | 5 g |
| - NaHCO3 | 0,35 g |
| - L-Glutamine | 0,75 g |
| - HEPES | 5,95 g |
| - D(+) glucose | 2,50 g |
| - H20 | Q.S. 800 ml |
| - Milieu 199 H modifié® | |
| - (x10)** | 4 ml (5%) |
| Additifs | |
| - Hémine bovine | 0,009 mM |
| - Glutathion réduit | 0,08 mM |
| - Solution de vitamines (x100) | 2 % |

On additionne à 1000 ml du milieu MA1, de la L-cystéine ( 3 mM ) et de l'acide bathocuproïne disulfonique ( 0,01 mM).

Le milieu MA1m ( m pour modifié ) est le milieu mA1 sans L-cystéine et sans acide bathocuproïne disulfonique, d'autre part, l'hémine bovine a été remplacée par de l'hémine porcine irradiée à 25 kilogray à une concentration nettement plus basse ( 0,003 mM ).

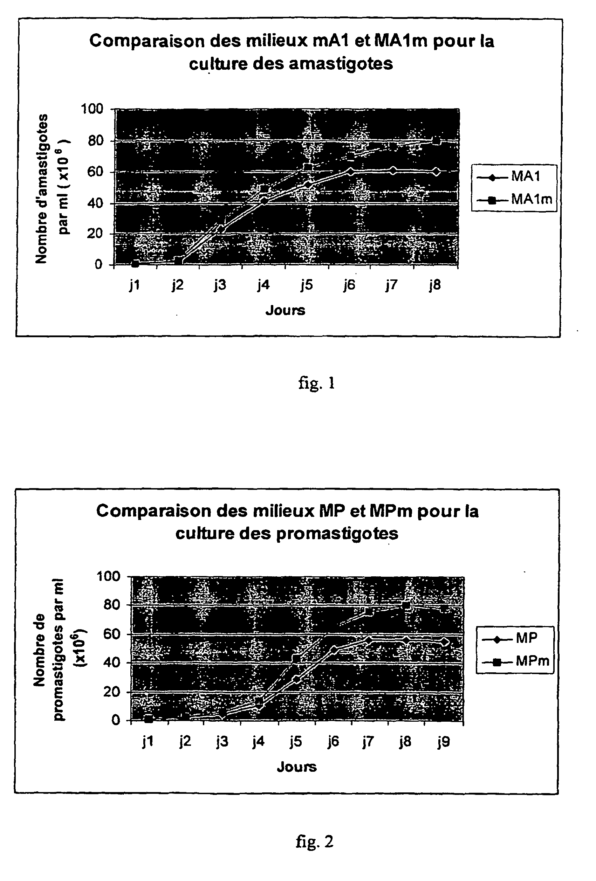

Les milieux MA1 et MA1 m ont été ensemencés avec une souche de Leishmania infantum MON1 et une comparaison de la croissance des amastigotes est effectuée dans le temps ( voir fig 1 ).

• Pour la culture des promastigotes, une baisse de la concentration de 2 composants ( RPMI et hémine ) ainsi que l'addition d'un antibiotique (gentamicine) constituent la principale modification du milieu de référence.

Il faut ajouter que l'hémine bovine a été remplacée par de l'hémine porcine irradiée à 25 kilogray comme dans le cas de la modification du milieu pour amastigotes.

| Milieu MP pour promastigotes | | Milieu MPm ( modifié ) pour promastigotes | |
|---|---|---|---|
| RPMI 1640 ( 1,1x ) | 1000 ml | RPMI 1640 ( 1x ) | 1000 ml |
| Avec L gluthamine et | | Milieu 199 H modifié 10 x | 2 % |
| Hepes | | Hémine porcine irradiée | 0,0002% |
| Milieu 199 H modifié (10 x) | 2 % | Gentamicine sulfate | 0,04 mg |
| Hémine bovine | 0,0005 % | | |

Les milieux MP et MPm ont été ensemencés avec une souche de Leishmania infantum MON1 et une comparaison de la croissance des promastigotes est effectuée dans le temps ( voir fig 2 ).

Le complexe obtenu selon l'invention comprend des molécules naturellement excrétées par les promastigotes et/ou amastigotes de Leishmanies sp., ainsi qu'un adjuvant qui induit de préférence une réponse à médiation cellulaire.

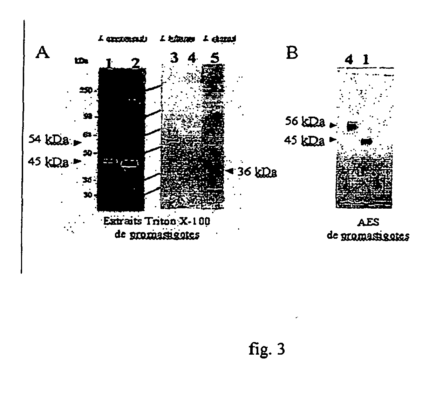

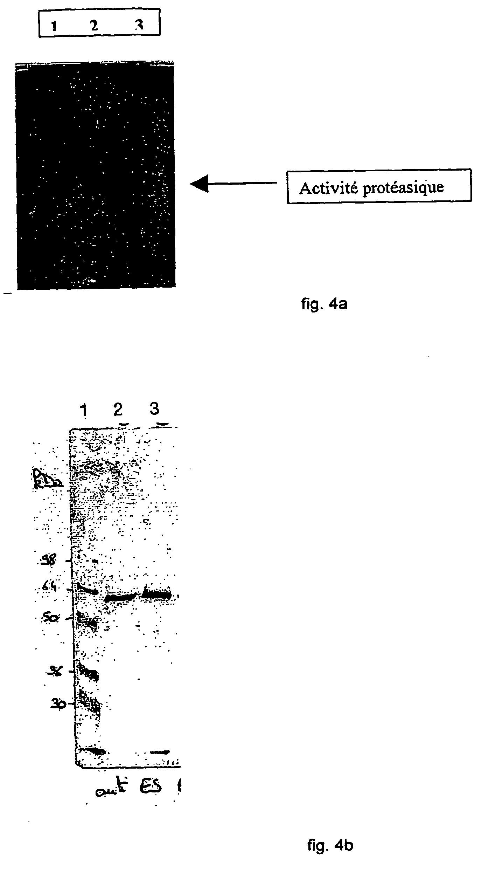

Ces molécules présentent au moins un épitope commun porté par une ou plusieurs protéines majeures. Leur poids moléculaire varie de 32Kda à 200Kda selon les espèces de leishmanies et en fonction du stade parasitaire considéré (fig. 3 : détection d'un épitope commun chez les diverses espèces de Leishmanies par l'anticorps monoclonal F5. A : extraits Triton x 100 de promastigotes, 1 et 2 : L. amazonensis (45 kDa), 3 et 4 : L. infantum (54 kDa), 5 : L. chagasi (36 kDa), B: AES de promastigotes). Ces molécules natives exprimant des activités protéasiques (fig 4a : activité protéasique (gel d'électrophorèse comportant de la gélatine), 1 = témoin complexe vaccinal, 2 et 3 = complexe vaccinal étudié) non répertoriées (ni metallo, ni sérine, ni cystéine protéase) sont dépourvues de tout contaminant sérique ou cellulaire.

Les formes promastigotes ou amastigotes sont cultivées en milieu axénique et asérique complètement défini selon le procédé décrit dans le Demande de Brevet d'invention WO 94/26 899 précitée et la modification apportée par la demanderesse.

Les cultures sont inoculées à raison de $5.10^5$ parasites par millilitre de milieu de culture. Les parasites, après incubation, sont éliminés par filtration tangentielle contre une membrane de 0,16 µ en polyéthersulfone et le filtrat est concentré 100 fois par filtration tangentielle contre un filtre de 3 kDa en polyéthersulfone.

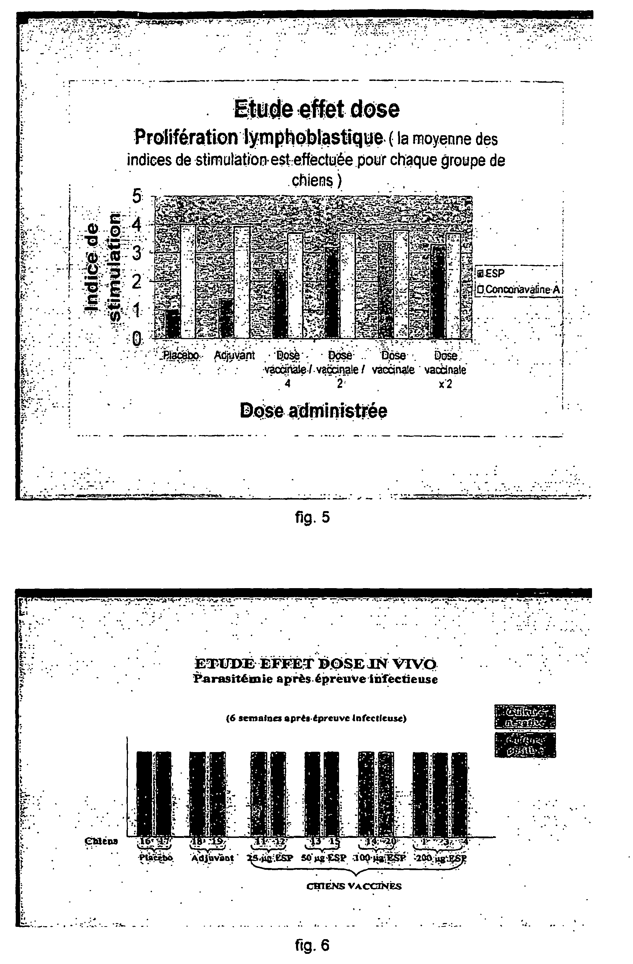

Chaque dose lyophilisée, établie après étude effet dose ( fig 5, 6, et 7 ), est constituée d'un lyophilisat de 100 µg de protéines d'excrétion sécrétion de Leishmanies et d'un diluant composé de 1 ml de sérum physiologique stérile.

La composition ainsi obtenue est administrée au mammifère infecté en présence d'un adjuvant, de préférence le muramyl dipeptide.

De façon préférentielle, le rapport protéine/adjuvant est compris entre 1/0,5 et 1/4.

Les études effectuées chez le chien ont permis de déterminer la dose vaccinale optimale à 200 µg de muramyl dipeptide avec un début de réponse dès 100 µg de protéines infectées.

Le mécanisme d'action spécifique du complexe vaccinal obtenu selon l'invention est vérifié à l'aide de méthodes classiques permettant le dosage des protéines, leur identification et la mesure de leur activité protéasique (techniques du Western Blot ou immunoblotting et SDS-PAGE) et à l'aide de méthodes plus spécifiques démontrant que le complexe vaccinal thérapeutique innovant agit soit par immunostimulation du système lymphocytaire de type Th1, soit par immunomodulation d'un type Th2 vers un type Th1.

Le procédé Western Blot permet la détection individuelle de protéines notamment des protéines excrétions sécrétions amastigotes (ESA) et excrétions sécrétions promastigotes (ESP) par réaction Antigène/Anticorps avec les immunsérums correspondants.

Pour chaque mammifère étudié (chien par exemple), une analyse sérologique est effectuée avec les ESA et ESP.

Les protéines du complexe vaccinal sont d'abord séparées par électrophorèse discontinue en gel de polycrylamide (PAGE) en présence de sodium dodecyl sulfate (SDS). Cette séparation est suivie d'un transfert électrophorétique des protéines sur une membrane de nitrocellulose selon le procédé de Towbin et al (Proc. Nath. Acad.Sci, 1979, 76, 4350-4354). Ces protéines sont ensuite détectées par réaction immunoenzymatique au moyen d'un anticorps monoclonal anti-ESP (fig 4b : Werstern Blot obtenu avec un anticorps monoclonal anti-AES de promastigotes, 4b1 : Protéines marqueur (kDa), 4b2 : Lot ESP à contrôler, 4b3 : Lot ESP de référence).

Un examen parasitologique est effectué à partir d'échantillon prélevé directement sur le candidat étudié, chien par exemple.

Un frottis à partir de ponction de moelle osseuse est effectué sur lame. Ce frottis, une fois fixé au méthanol est coloré au May Grümwald Giemsa et observé au microscope à immersion (x 1000).

Des prélèvements de moelle osseuse sont mis en culture dans le milieu de culture biphasique NNN (Novy and Mac Neal, 1904, J. Infec. Dis., 1 :1-30) dont du RPMI 1640 additionné de 20% de sérum de veau foetal décomplémenté constitue la phase liquide. Des repiquages à l'aveugle sont réalisés tous les quatre à six jours. Les cultures sont régulièrement observées en microscopie photonique (X 400) pendant 20 mn.

Les parasitémies sont quantifiées comme suit :

+/- : formes allongée réfringente immobile ;
+ : 1 à 5 formes promastigotes mobiles/champs ;
++ : > 5formes promastigotes mobiles/champs;
+++ : - culture à confluence.

Mise en évidence de l'implication d'une immunité à médiation cellulaire de type Th1 :

Des Leishmanies de formes promastigotes sont cultivées dans des milieux de culture définis selon les méthodes décrites précédemment. Des parasites de fin de phase exponentielle (6-7 jours) sont récoltés. Le culot parasitaire est lavé trois par centrifugation (2500 g, 15 mn, 4°C) en tampon PBS. Après avoir vérifié la viabilité des parasites à l'aide d'un colorant vital (le bleu Trypan), une suspension contenant $2 \times 10^8$ parasites par ml est inactivée en tampon PBS contenant 0,01 % de merthiolate (Pinelli *et al.*, 1994, Infect. Immun., 62 : 229-235). Ceci constitue les leishmanines pour le test d'intradermoréaction ( JDR).

L'étude de la réponse immunitaire de type Th1 ci-après est effectuée sur des chiens.

Les chiens sont placés en décubitus latéral et une tonte délicate et non irritante est effectuée sur zone thoracique d'environ 5 cm sur 10 cm en arrière du coude. Quatre cercles de 10 mm de diamètre sont délimités à l'aide d'un feutre.

On injecte, au centre des cercles 0,1 ml de solution en intradermo-injection. Deux cercles reçoivent de la solution de leishmanines et les deux autres cercles reçoivent de la solution saline merthiolée en contrôle négatif. La lecture de l'intra Dermo Réaction (IDR) s'effectue 48 heures plus tard au moyen d'une réglette d'allergologue.

Le test est considéré positif si la moyenne de deux diamètres d'induration observée est supérieure ou égale à 5 mm. L'observation d'un érythème sans induration sera considérée comme un test négatif (Pinelli *et al.,* 1994, Infect. Immun., 62 : 229-235 ; Marty *et al.,* 1994, Trans. Roy. Soc. Trop. Med. Hyg., 88, 658-659).

On effectue ensuite un test de prolifération lymphocytaire.

Les cellules mononuclées du sang périphérique (PBMC) des chiens sont séparées sur gradient de Ficoll (densité 1, 078) par centrifugation à 800 g pendant 20 mn à température ambiante. Ces cellules sont mises en culture en plaque de 96 puits à la concentration de $2.10^5$ cellules par puits en présence de 2 μg par ml de Concanavalin A (Sigma), de 5 μg par ml de ES P ou 20 ml de sumageants de culture récoltés en phase stationnaire de la croissance des promastigotes (SP) par puits, et en absence de tout additif dans un volume de 200 ml de milieu RPMI 1640 contenant 5 % de sérum de veau foetal décomplémenté, 2mM de L-glutamine, 100 U de pénicilline par ml, 100 mg de streptomycine par ml. Les concentrations optimales d'antigènes et de mitogène ont été déterminées dans des expériences préalables. Les PBMC sont incubées pendant 72 heures dans une atmosphère humide à 37°C en présence de 5% de $CO_2$ puis pendant 20 heures avec 0, 5 μCi de $^3H$ thymidine. Les cellules sont récoltées sur filtre et l'incorporation de la radioactivité est déterminée par comptage en liquide scintillant (compteur β). Tous les tests sont réalisés en triplicata.

Une méthode immunohistochimique plus rapide et plus sensible utilisant le BrdU (5-bromo-2'-désoxyuridine), une analogue structurale de la thymidine est aussi utilisée pour mesurer la prolifération cellulaire (BrdU, cell proliferation détection kit III, Boehringer Mannheim, Allemagne). Dans nos expériences, le BrdU est additionné pendant 18 heures après 72 heures d'incubation. Les cellules qui ont incorporé le BrdU dans leur ADN sont facilement détectables en présence d'un anticorps monoclonal dirigé contre le BrdU.

Les réponses prolifératives sont exprimées en indices de stimulation qui représentent le rapport de la moyenne de prolifération après stimulation sur la moyenne de prolifération en absence d'antigène.

La prolifération lymphocytaire a aussi été estimée par des lectures visuelles en microscopie photonique (- : négatif; +/- : légère prolifération ; + : petite prolifération en moins de 5 points par champs microscopique ; ++ : prolifération moyenne en plus de 5 points ; +++ : forte prolifération).

Le titrage de l'activité leishmanicide des monocytes est effectué selon la méthode LEMESRE décrite ci-dessous.

Pour ce test, les monocytes et les lymphocytes sont isolés à partir de sang veineux des chiens. Les monocytes sont mis en culture pendant 3 jours à raison de $10^5$ cellules par puits dans des chambres de culture (Labteck) dans un milieu RPMI 1640 complet (contenant 25 mM d'HEPES, 2 mM de L-glutamine, 100 U de pénicilline par ml, 100 mg de streptomycine par ml et 10 % de sérum de veau foetal inactivé) à 37°C dans une atmosphère humide contenant 5% de $CO_2$. Après 3 jours de culture, les macrophages sont lavés en milieu RPMI complet, additionnés de milieu frais et mis en contact avec des formes promastigotes métacycliques de *L. infantum* dans un rapport de 5 parasites par cellules, à 37°C pendant une nuit ou 5 heures selon les expériences. Les macrophages sont alors lavés avec du milieu RPMI complet frais pour éliminer les parasites non phagocytés. Les cellules sont mises en incubation soit seules, soit en présence de 5 $\mu$g d'antigènes ESP, soit en présence de lymphocytes autologues, soit en présence de sumageants de co-culture de macrophages infectés et de lymphocytes autologues et de témoins correspondants (récoltés à 5 heures) et ceci à 37°C dans une atmosphère humide à 5% de $CO_2$ pour une durée de 48 heures. Lorsqu'ils sont utilisés, les lymphocytes cultivés séparément sont lavés, comptés et additionnés aux macrophages dans la proportion de 2 lymphocytes par macrophage.

Après 48 heures d'incubation, les cellules sont lavées trois fois en tampon PBS 0,01 M, pH 7,2, fixées au méthanol puis colorées au Giemsa. L'activité leishmanicide des macrophages est estimée en microscopie photonique (1000X) en déterminant le pourcentage de macrophages infectés et le nombre de formes amastigotes intactes pour 100 cellules (2 fois 200 cellules sont observées en duplicata). Les résultats sont exprimés en pourcentage d'inhibition de l'index parasitaire = 100 - (IP x 100).

IP= Index parasitaire = [(le nombre moyen d'amastigotes par macrophage dans l'échantillon traité) x (le pourcentage moyen de macrophages infectés dans l'échantillon traité)] / [(le nombre moyen d'amastigotes par macrophage dans l'échantillon témoin) x (le pourcentage moyen de macrophages infectés dans l'échantillon témoin)].

On peut également procéder au dosage du monoxyde d'azote (NO) pour connaître l'activité destructrice des monocytes à l'encontre des Leishmanies. La synthèse de NO par les monocytes est en effet signe de destruction des leishmanines par les monocytes ayant été activés par les cytokines de type interferon gamma (IFN$\gamma$)

Le NO possède une haute réactivité chimique. En présence d'eau et d'oxygène, cette molécule est rapidement oxydée de façon stochiométrique et forme ainsi des nitntes (NO2-) selon la réaction :

$$4 \text{ NO}° + O_2 + 2H_2O \text{ --- } 4 \text{ NO}_2^- + 4 \text{ H}^+$$

Les nitrites s'accumulent dans les milieux et sont facilement détectables chimiquement par la méthode de Griess.

A 50 $\mu$l de surnageant à tester, on ajoute 60 $\mu$l de Griess A (Sulfanilamide 1% dans HCl 1,2N) et 60 $\mu$l de Griess B (N-(1-Naphtyl)ethyl-enediamine 0,3 %). La réaction colorimétrique se développe à l'abri de la lumière pendant 2 minutes. Les densités optiques obtenues à 540 nm sont corrigées par la soustraction des DO obtenues sur les puits contenant le milieu de culture seul.

Les valeurs obtenues sont reportées sur une courbe étalon (DO = f($NO_2^-$)) réalisée à partir de concentrations connues de $NO_2^-$.

Le tableau ci-après montre les réponses sérologiques obtenues lors de nos expériences et le suivi de la parasitémie (analyses effectuées 2 mois et 8 mois après l'épreuve infectieuse).

| | | SEROLOGIE | | | | PARASITEMIE (sur ponction de Moelle) | |
|---|---|---|---|---|---|---|---|
| | Chiens | IF quantitative | WB (ESA) | WB (ESP) | ELISA (IgG2) ESA/ESP | Examen direct | Culture sur milieu NNN |
| Chiens Témoins | MUMA | - | - | - | - | + | ++ |
| | LEO | - | - | - | - | + | ++ |
| Chiens Immunisés ESA | LOUBARD | 1/200 | + | + | + (0,700) | - | - |
| | MINA | 1/200 | ± | ± | + (0,450) | - | - |
| Chiens Immunisés ESP | NOUGAT | 1/800 | + | + | + (0,780) | - | - |
| | MINON | 1/100 | ± | ± | + (0,520) | - | - |

*Légende :*

IF : Immunofluorescence (considérée positive si le titre est ≥ 1/100)

WB : Western Blot

ELISA : Cutt off = 0,300 en DO (densité optique)

Parasitémie : culture sur milieu NNN

- = absence

++ = plus de 5 formes promastigotes mobiles/champs

Le tableau suivant montre les réponses obtenues de type cellulaire et le rôle inhibiteur des serums sur la prolifération parasitaire (analyses effectuées 2 mois après l'épreuve infectieuse).

| | Chiens | IDR | REPONSES A MEDIATION CELLULAIRE | | | POURCENTAGE D'INHIBITION DE LA PROLIFERATION DES LEISHMANIES | |
| --- | --- | --- | --- | --- | --- | --- | --- |
| | | | Test de prolifération lymphoblas-tique | Activité leishmani-cide des monocytes | Dosage du NO (en µM) | Prolifération des promasti-gotes | Prolifération des amasti-gotes |
| Chiens Témoins | MUMA | + | + 2,1 (3) | 15,5% | 0,3 | 20% | 15% |
| | LEO | - | + 1,2 (3,1) | 21,5% | ND | ND | ND |
| Chiens Immunisés ESA | LOUBARD | + | ++ 2,9 (3,2) | 58,9% | ND | 50% | 41% |
| | MINA | + | ++ 3,8 (4,2) | 47,8% | ND | 69% | 52% |
| Chiens Immunisés ESP | NOUGAT | + | ++ 3,1 (4,2) | 75,6% | 3,9 | 98% | 54% |
| | MINON | + | +++ 3,5 (4,5) | 64,1% | 2,8 | 72% | 56% |

Légende :

- IDR : Le test Intra Dermo Réaction est considéré positif (+) si l'induration est ≥ 5 mm 48 h après l'intradermoinjection

- Test de prolifération lymphoblastique : Les résultats sont exprimés par lecture en microscopie photonique et en indices de stimulation (entre parenthèses, indice de stimulation du témoin + Concanavaline A)

+ : petite prolifération    ++ : moyenne prolifération        +++ : forte prolifération

- Activité leishmanicide des monocytes : exprimée en pourcentage d'inhibition de l'index parasitaire

- Dosage du NO :

- Rôle inhibiteur des sérums : résultats exprimés en pourcentage d'inhibition de la croissance

ND= Non Déterminé

Rôle inhibiteur des anticoms anti-facteurs d'excrétion sécrétion sur le développement parasitaire de L. infantum :

Ces tests visent à montrer l'éventuel effet inhibiteur des anticorps anti-ES sur la prolifération et la différenciation *in vitro* des parasites.

100 µl d'immunsérum préalablement inactivé (56°C pendant 45 minutes) de différents groupes de chiens sont mis en contact pendant trente minutes à température ambiante avec 5 x 10[6] formes promastigotes métacycliques. Des tests de viabilité avant et après traitement (cf ci dessus) sont réalisés pour établir les pourcentages de mortalité. Les parasites ainsi traités sont mis en culture, soit à 25°C dans le milieu RPMI 1640 contenant 10 % SVF (Serum Veau Foetal), soit à 37°C dans le milieu MAA/20 (10[6] parasites par ml de milieu). Les cinétiques de prolifération des formes promastigotes ainsi que celles des amastigotes sont établies par comptage journalier des cellules en microscopie photonique. Les résultats sont exprimés en pourcentage d'inhibition de la croissance.

Le caractère innovant du complexe vaccinal selon l'invention ne réside pas seulement dans l'induction d'une réponse cellulaire spécifique de type Th1 mais également dans la production de faibles taux d'anticorps très efficaces envers les promastigotes et les amastigotes de Leishmania.

Pour le déroulement des études, d'autres techniques particulières ont été utilisées.

Méthode d'épreuve infectieuse

L'épreuve infectieuse consiste à injecter en intraveineuse $10^6$ promastigotes en phase métacyclique traités au complément de chien sain et de $5.10^6$ macrophages péritonéaux de chien sain, infectés in vitro par des amastigotes. Les promastigotes et les macrophages infectés sont dilués dans du sérum physiologique stérile pour un volume final de 1,5 ml. Ce mélange est effectué juste avant l'injection.

Détection des immunoglobulines de type G2 (IgG2) de chiens, spécifiques des ES

Cette détection s'effectue par la méthode Western Blot en utilisant un conjugué anti IgG2 (Immunoglobulines G2) de chien et par méthode ELISA selon la technique de microtitration de Kweider et al (J.Immunol, 1987, 138, 299).

Le complexe vaccinal selon l'invention peut être administré de diverses manières. Il est cependant administré de façon préférentielle par 4 voies :

- Soit par injection sous cutanée
- Soit par injection intra dermique
- Soit par injection intra musculaire
- Soit par voie orale

D'autres modes d'administration peuvent être employés comme la voie parentérale ou intraveineuse.

De façon générale, un vaccin se présente sous une forme injectable composée d'une fraction lyophilisée que l'on reprend par une fraction liquide ou diluant. Les doses utilisées pour la prévention et l'immunothérapie sont différentes, et sont également différentes selon le mode d'injection :

**• par voie sous cutanées et intra musculaires**

- Injection d'une dose (100 $\mu$g de protéines excrétées secrétées et 200 $\mu$g d'adjuvant) chez les chiens quelle que soit la race, l'âge et le sexe pour un effet préventif).
- Injection de demi doses (50 $\mu$g de protéines excrétées secrétées et 100 $\mu$g d'adjuvant) pour l'immunothérapie des chiens leishmaniens.

**• par voie intra dermo**

- Injection de demi dose chez les chiens pour un effet préventif.
- Injection de quart de dose chez les chiens leishmaniens pour un effet thérapeutique.

Les méthodes d'injections sont reprises dans les exemples d'immunothérapie et de vaccination ainsi que dans les études d'innocuité.

Les études d'innocuité sur le complexe vaccinal ont été effectuées sur 30 chiens.

Tous les chiens sont des beagles adultes de 1 an à 6 ans, 50% mâles et 50% femelles provenant de zone non endémique. Ces chiens sont parfaitement sains, présentent une sérologie et un test Intra Dermo Réaction (IDR) négatifs vis à vis de Leishmania.

Parmi ces chiens certains reçoivent des placebos. Parallèlement au suivi clinique, un suivi du statut immunitaire spécifique vis à vis du complexe vaccinal est effectué (démonstration de l'induction de l'immunité à médiation cellulaire et humorale de type Th1 uniquement chez les chiens vaccinés).

Déroulement des essais

Les essais ont été effectués en BPL (Bonnes Pratiques de Laboratoire) et en BPC (Bonnes Pratiques Cliniques).

(Primo vaccination) → 4 semaines → Dose → 4 semaines → Surdose → 4 semaines
d'observation    réitérée   d'observation    (2 doses    d'observation
simultanées)

Les doses sont injectées par voie sous cutanée.

Un suivi de la tolérance est effectué :

Après administration, un examen visuel direct : douleur, tuméfaction, chaleur et prurit est effectué quotidiennement au point d'injection pendant 14 jours.

Un suivi de la tolérance générale est également effectué. Il s'agit d'un examen rapide journalier avec prise de température, un examen clinique vétérinaire hebdomadaire incluant une palpation ganglionaire (poplité), une palpation abdominale, le suivi d'arthrites et d'uvéites et une pesée.

Les suivis hématologiques et biochimiques (créatinine, urée, transaminases) sont effectués 3 semaines après chaque injection vaccinale.

Résultats d'innocuité

Parmi les 30 chiens, qui comportent 7 placebos, aucun trouble général n'a été observé. Seules quelques réactions locales mineures sont à signaler : léger oedème au point d'injection, erythème modéré et léger prurit. Ces troubles sont bénins et rétrocèdent spontanément en 24 à 48 h. Ils sont tout à fait logiques pour un vaccin à médiation cellulaire.

Aucune anomalie n'est à noter au niveau des suivis hématologiques et biochimiques. Des résultats similaires ont été obtenus après injection intra dermiques chez 5 chiens et après injection intra musculaires chez 5 chiens.

Le complexe vaccinal ne présente donc aucun problème d'innocuité.

Activation spécifique de lymphocytes T de type Th1

Parallèlement au suivi sérologique par immunofluorescence classique utilisant des lames coatées par des promastigotes (méthode de référence sérologique pour la leishmaniose canine) qui se révèle faible chez tous les chiens, l'étude de la réponse à médiation cellulaire par test de prolifération lymphoblastique et par étude de l'activité leishmanicide des monocytes, est effectuée sur les 30 chiens.

Les 7 placebos n'induisent pas une réponse cellulaire spécifique de l'antigène vaccinal, par contre les 23 chiens vaccinés présentent bien une induction du système Th1 avec notamment des index de prolifération lymphocytaires spécifiques du complexe vaccinal comparables à l'index témoin (Concanavalline A) qui s'accompagnent de pourcentages d'inhibition de l'index parasitaire élevés (> 40% avec une moyenne de 60% sur 23 chiens vaccinés).

Le complexe vaccinal a donc pour effet d'activer les monocytes envers les leishmania par l'intermédiaire des lymphocytes tout en ayant aucun effet sur le système Th2.

Etude effet dose du complexe vaccinal

Cette expérience a pour but de déterminer la dose vaccinale minimale qui induit une réponse Th1 efficace.

Pour cela, 12 chiens beagle adultes de 1 à 6 ans de zone non endémique sont répartis en 6 groupes de 2 :

1$^{er}$ groupe: placebo
2$^{ème}$ groupe : placebo + 200 $\mu$g d'adjuvant
3$^{ème}$ groupe : 25 $\mu$g protéines excrétées secrétées et 50 $\mu$g d'adjuvant
4$^{ème}$ groupe : 50 $\mu$g protéines excrétées secrétées et 100 $\mu$g d'adjuvant
5$^{ème}$ groupe : 100 $\mu$g protéines excrétées secrétées et 200 $\mu$g d'adjuvant
6$^{ème}$ groupe : 200 $\mu$g protéines excrétées secrétées et 400 $\mu$g d'adjuvant

Ces essais sont effectués en BPL et en BPC.

| Primo | 4 semaines | 2<sup>nde</sup> injection | 4 semaines | Epreuve |
|---|---|---|---|---|
| Vaccination | | | | infectieuse |

L'épreuve infectieuse consiste à infecter les chiens par voie intra veineuse à l'aide de promastigotes en phase métacyclique et de monocytes infectés d'amastigotes.

Suite à la 2<sup>nde</sup> injection, l'étude de l'état immunitaire permet d'affirmer que les chiens ayant reçu le complexe vaccinal sont bien en Th1 avec un début de réponse maximale en plateau à partir de 50 $\mu$g de protéines secrétées excrétées injectées. Ce phénomène de plateau s'observe aussi bien pour le test de prolifération lymphocytaire que pour l'activité des monocytes.

Le graphique de la figure 5 montre les résultats donnés par l'étude effet-dose : étude de la prolifération lymphoblastique selon la dose vaccinale injectée.

D'autre part, une étude parasitémique sur ponction de moelle est effectuée 2 mois après l'épreuve infectieuse en utilisant le milieu de culture de référence NNN (Novy and Mac Neal, J.infect, Dis, 1904, 1, 1-30).

Les 4 chiens placebos et un chien ayant reçu 50 $\mu$g de protéines excrétées secrétées présentent une parasitémie positive.

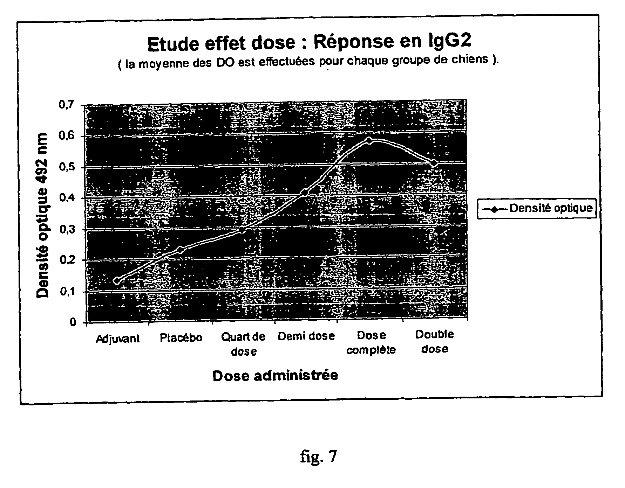

Le graphisme de la figure 6 montre les résultats donnés par l'étude effet-dose : étude de la parasitémie, 6 semaines après l'épreuve infectieuse selon la dose vaccinale injectée.

Anticorps spécifiques liés au système Th1

Comme cela a été précédemment démontré, le système Th1 correspond à une réponse à médiation cellulaire avec une activation des macrophages via les lymphocytes producteurs de cytokines spécifiques. C'est le rôle principal du complexe vaccinal selon l'invention. Cette réponse cellulaire s'accompagne d'une faible réponse humorale que nous pouvons démontrer aisément par la méthode classique d'immunofluorescence en utilisant un conjugué anti IgG total marqué à la fluorescéine.

Néanmoins certains travaux préliminaires chez l'homme (KAWANO.P et al, Parasite Immunol, 1995, 17, 451-458) et chez le chien (NIETO C.G et al, Vet Immunol and Immunopathology, 1999, 67, 117-130) démontrent que les isotypes d'IgG seraient des marqueurs de la dichotomie immunitaire Th1/Th2. Plus précisément, un chien atteint de leishmaniose avec des signes cliniques probants présente un taux élevé d'anticorps principalement d'isotype IgG1 alors qu'un chien asymptomatique présente des anticorps spécifiques d'isotype IgG2. Les chiens ayant reçu le complexe vaccinal selon l'invention présentent des taux faibles d'IgG2 spécifiques des protéines secrétées excrétées, ce qui est conforme à l'expansion préférentielle de lymphocytes T de type Th1.

Le graphique de la figure 7 montre la réponse spécifique des chiens vaccinés en IgG2 envers le complexe vaccinal objet de l'invention selon la dose vaccinale injectée (méthode ELISA sur cupules).

**RESULTATS EN IMMUNOTHERAPIE**

Selon les spécialistes comme PINELLI (PINELLI.E et al, Infect Immun, 1994, 62,229-235) les chiens leishmaniens correspondant à l'activation du système lymphocytaire de type Th2 présentent une réponse en anticorps élevée.

Cette production accrue en anticorps correspond à l'hyperprotéinémie et induit l'apparition d'immuncomplexes entraînant une atteinte rénale (augmentation de la créatinine et de l'urée sanguines).

Lors des études et essais, on a donc essayé de moduler vers un état Th1 en administrant à des chiens parfaitement leishmaniens par voie intra musculaire des doses du complexe vaccinal. Le suivi de l'état immunitaire ainsi que l'observation clinique sont effectués avant et après traitement.

**Exemple 1 : chien LOYD**

Un chien mâle de race Epagneul Breton LOYD, âgé de 6 ans appartenant à Mme C présente de très nombreuses lésions cutanées accompagnées d'un état de fatigue général et un aspect maigre, le tout évoquant une leishmaniose canine. LOYD vit près d'Aix-en-Provence en pleine zone endémique et passe la majorité de son temps à l'extérieur. C'est donc un animal prédisposé à être piqué par les phlébotomes.

Ces lésions cutanées sont de plusieurs types : pustules et papules au niveau du chanfrein ; érythème sur le flanc et dans la face interne des oreilles ; prurit, squames et croûtes au niveau des coudes.

Le vétérinaire, le Dr DM diagnostique un pemphigus foliacé accompagné d'une leishmaniose. Ce dernier diagnostic est confirmé par une observation directe au microscope de leishmanies à partir d'un calque cutané et une analyse sérologique qui donne un titre en immunofluorescence leishmaniose positif à 1/1600.

Pendant 8 mois, un traitement classique aux sels d'antimoine et aux corticoïdes s'avère négatif. On a donc instauré une immunothérapie qui consiste à effectuer par voie intramusculaire 4 injections de 50 μg de complexe vaccinal (1/2 dose), chaque injection étant espacée de 10 jours.

L'analyse de l'état immunitaire avant toute injection permet d'affirmer que le chien est bien dans un état immunitaire de type Th2 avec un titre en anticorps élevé ainsi que des tests de prolifération lymphoblastiques et activation monocytaire négatifs.

Une semaine après la seconde injection, le chien LOYD retrouve de l'appétit et une certaine vitalité. Le Dr DM commence à observer une légère amélioration cutanée.

Un mois après la dernière injection, LOYD a retrouvé un aspect clinique normal avec notamment une augmentation de poids de 1 kg et une disparition à 80% de toutes lésions cutanées. L'analyse de l'état immunitaire permet d'affirmer une baisse du titre en anticorps anti leishmania qui descend à 1/400 en immunofluorescence. Parallèlement, les monocytes ont retrouvé une activité leishmanicide (avec un pourcentage d'inhibition de l'index parasitaire égal à 75%) et le test de prolifération lymphoblastique est parfaitement positif.

Une recherche des parasites par culture sur milieu NNN s'avère négative. 8 mois après le traitement, le chien LOYD présente parfois des lésions sur le chanfrein qui correspondent au pemphigus foliacé, lésions disparaissant après un traitement corticoïde. Les analyses biologiques permettent d'affirmer que LOYD est toujours en état immunitaire Th1.

**Exemple 2 : Chien JAZZ**

Un chien mâle de race rottweiler, JAZZ, âgé de 5 ans appartenant à Mr C présente les signes cliniques spécifiques de la leishmaniose. Selon le Dr GH : présence de nombreuses squames brillantes, dépilation périoculaire droite, lésions ulcéreuses au niveau des 2 coudes antérieurs et un état de fatigue prononcé. Les analyses biologiques avec notamment une sérologie leishmaniose positive au 1/400 en immunofluorescence confortent le diagnostic clinique.

Une immunothérapie consistant à effectuer par voie intramusculaire 3 injections de 50 μg de complexe vaccinal (1/2 dose), chaque injection étant espacée de 10 jours, est instaurée. L'analyse de l'état immunitaire avant toute injection démontre que le chien JAZZ développe un système immunitaire de type Th2 avec une parasitémie fortement positive à partir de la moelle osseuse.

Un mois après la dernière injection, les signes cliniques leishmaniens de JAZZ rétrocèdent avec notamment une cicatrisation des lésions ulcéreuses, une disparition importante des squames ainsi qu'une dépilation périoculaire presque inexistante. La sérologie présente toujours un titre en immunofluorescence égal au 1/400. Par contre, l'analyse de la réponse cellulaire permet d'affirmer que JAZZ présente un état Th1 actif avec un test de prolifération lymphoblastique positif et une activité leishmanicide intramacrophagique élevée.

Parallèlement, la parasitémie s'est négativée (culture de moelle osseuse en milieu NNN).

La présente invention consiste donc bien à un complexe vaccinal et thérapeutique qui induit le passage d'un état immunitaire de type Th2 avec production importante d'anticorps qui exacerbe les manifestations cliniques vers un état immunitaire de type Th1 entraînant la guérison.

**RESULTATS EN VACCINATION**

Afin d'évaluer l'efficacité du complexe vaccinal selon l'invention, le complexe vaccinal est testé sur 6 chiens parfaitement sains. Ces 6 chiens présentent une sérologie leishmaniose négative, une parasitémie négative ainsi que des tests de réponse cellulaire spécifique à Leishmania parfaitement négatifs.

Ces 6 chiens vivent dans un endroit exempt de tout phlébotome. Nous définissons 3 groupes de chiens, chaque groupe comportant un mâle et une femelle.

• Groupe témoin (Placebos)

- *Témoin négatif :* Chien LEO de race Pointer, sexe mâle. Age 3 ans
- *Témoin adjuvant seul:* Chienne MUMA de race Epagneul Breton, sexe femelle. Age : 6 ans

• Groupe chiens vaccinés avec protéines d'excrétion secrétions de promastigotes (ESP)

- Chienne MINON de race Braque de Weymar, sexe femelle. Age : 2 ans et demi
- Chien NOUGAT de race Pointer, sexe mâle. Age : 2 ans et demi

• Groupe chiens vaccinés avec protéines d'Excrétion Secrétion d'Amastigotes (ESA)
- Chien LOUBARD de race Epagneul Breton, sexe mâle. Age : 4 ans

- Chienne MINA de race Braque de Weymar, sexe femelle : Age : 3 ans

Le schéma d'injection vaccinale est le suivant :

J0                        J28                          J84

$1^{ère}$ injection                 $2^{nde}$ injection

1 dose en sous → 4 semaines → 1 dose en sous → 8 semaines → épreuve

cutanée                       cutanée               infectieuse

Un suivi clinique des 6 chiens est effectué toutes les deux semaines. Les analyses biologiques sont schématisées de la façon suivante :

J0_____J28_____J84_____J84

$1^{ère}$ injection    ↓    $2^{nde}$ injection      ↓      épreuve      + 2mois

             J14                 J56     infectieuse        ↓

    Analyses biologiques      Analyses biologiques      Analyses biologiques

Les analyses biologiques consistent en :

- analyses biochimiques : urée, créatine, transaminases
- analyses hématologiques : numération, formule
- sérologie leishmaniose : immunofluorescence quantitative anti Leishmania, par méthode Western Blot envers les antigènes d'excrétion secrétion et dosage par méthode ELISA des IgG2 spécifiques.
- tests de réponse cellulaire : Test de prolifération lymphoblastique, étude de l'activation des macrophages et test IDR (Intra Dermo Réaction), dosage du NO.
- Etude du rôle neutralisant des anticorps anti ES.

Il faut ajouter à ces analyses, la recherche des leishmania par observation directe au microscope et culture sur milieu NNN à partir de moelle osseuse après l'épreuve infectieuse.

Résultats :

*Suivi clinique :*

Aucune manifestation clinique importante n'est apparue pendant toute cette étude. Il faut noter un léger amaigrissement et l'apparition de quelques squames chez le chien LEO 2 mois après l'épreuve infectieuse.

*Suivi biologique :*

- Les paramètres biochimiques et hématologiques sont restés normaux tout au long de cette étude.
- Sérologie leishmaniose et parasitémie

Avant toute injection, les 6 chiens présentent des sérologies et parasitémies négatives. Le tableau ci-après montre les réponses sérologiques obtenues lors des expériences effectuées et le suivi de la parasitémie (analyses effectuées 2 mois et 8 mois après l'épreuve infectieuse).

| | | SEROLOGIE | | | | PARASITEMIE (sur ponction de Moelle) | |
|---|---|---|---|---|---|---|---|
| | Chiens | IF quantitative | WB (ESA) | WB (ESP) | ELISA (IgG2) ESA/ESP | Examen direct | Culture sur milieu NNN |
| Chiens Témoins | MUMA | - | - | - | - | + | ++ |
| | LEO | - | - | - | - | + | ++ |
| Chiens Immunisés ESA | LOUBARD | 1/200 | + | + | + (0,700) | - | - |
| | MINA | 1/200 | ± | ± | + (0,450) | - | - |
| Chiens Immunisés ESP | NOUGAT | 1/800 | + | + | + (0,780) | - | - |
| | MINON | 1/100 | ± | ± | + (0,520) | - | - |

*Légende :*

IF : Immunofluorescence (considérée positive si le titre est ≥ 1/100)

WB : Western Blot

ELISA : Cutt off = 0,300 en DO (densité optique)

Parasitémie : culture sur milieu NNN

- = absence

++ = plus de 5 formes promastigotes mobiles/champs

- Seuls les chiens immunisés présentent des anticorps spécifiques envers les ESA et ESP (Western Blot), des IgG2 spécifiques (ELISA) ainsi que des parasitémies négatives. Il faut noter une légère apparition d'anticorps totaux (1/200 en IF) chez tous les chiens après l'épreuve infectieuse.

Seuls les chiens témoins (LEO et MUMA) présentent des parasitémies positives ainsi qu'une absence d'anticorps spécifiques IgG2 anti ES.

• Réponse à médiation cellulaire

Avant toute injection, les 6 chiens présentent une réponse à médiation cellulaire envers Leishmania infantum parfaitement négative. Selon le tableau II, seuls les chiens immunisés présentent des tests de prolifération lymphoblastique, des activités leishmanicides intramacrophagiques positives associées à la production de NO par les monocytes.
Le tableau suivant montre les réponses obtenues de type cellulaire et le rôle inhibiteur des serums sur la prolifération parasitaire (analyses effectuées 2 mois après l'épreuve infectieuse).

| | Chiens | IDR | Test de prolifération lymphoblas-tique | Activité leishmani-cide des monocytes | Dosage du NO (en µM) | % d'inhibition de la prolifération des leishmanies | |
|---|---|---|---|---|---|---|---|
| | | | | | | Prolifération des promasti-gotes | Prolifération des amasti-gotes |
| Chiens Témoins | MUMA | + | + 2,1 (3) | 15,5% | 0,3 | 20% | 15% |
| | LEO | - | + 1,2 (3,1) | 21,5% | ND | ND | ND |
| Chiens Immunisés ESA | LOUBARD | + | ++ 2,9 (3,2) | 58,9% | ND | 50% | 41% |
| | MINA | + | ++ 3,8 (4,2) | 47,8% | ND | 69% | 52% |
| Chiens Immunisés ESP | NOUGAT | + | ++ 3,1 (4,2) | 75,6% | 3,9 | 98% | 54% |
| | MINON | + | +++ 3,5 (4,5) | 64,1% | 2,8 | 72% | 56% |

**Légende :**

- IDR : Le test Intra Dermo Réaction est considéré positif (+) si l'induration est ≥ 5 mm 48 h après l'intradermoinjection

- Test de prolifération lymphoblastique : Les résultats sont exprimés par lecture en microscopie photonique et en indices de stimulation (entre parenthèses, indice de stimulation du témoin + Concanavaline A)

  + : petite prolifération    ++ : moyenne prolifération    +++ : forte prolifération

- Activité leishmanicide des monocytes : exprimée en pourcentage d'inhibition de l'index parasitaire

- Dosage du NO :

- Rôle inhibiteur des sérums : résultats exprimés en pourcentage d'inhibition de la croissance

ND= Non Déterminé

- Etude du rôle neutralisant des anticorps anti ES :

Cette analyse effectuée sur 1 chien de chaque groupe (tableau II) permet d'affirmer que les chiens immunisés par les ES présentent des anticorps très efficaces inhibant aussi bien la prolifération des promastigotes que celles des amastigotes contrairement aux chiens témoins.

De par ces analyses, le complexe vaccinal induit bien une immunité à médiation cellulaire de type Th1 protecteur auquel il faut ajouter une induction d'anticorps spécifiques d'isotype IgG2 inhibant significativement la prolifération des leishmanies.

**Revendications**

1. Complexe vaccinal thérapeutique destiné à la prévention ou au traitement des leishmanioses et des infections à microorganismes pathogènes intracellulaires chez le mammifère et en particulier chez l'homme, les canidés, les félidés et les équidés, **caractérisé en ce qu'**il est composé

   - d'une part de molécules d'excrétion sécrétion issues d'amastigotes et/ou de promastigotes de Leishmania sp. produites dans un milieu axénique et asérique parfaitement défini tel que le milieu comprend :

   pour les amastigotes (quantités pour 800 ml):

   | | |
   |---|---|
   | - Milieu 199 H ® (x10) ( avec sels de Hanks ) | 100 ml |
   | - Trypto-caséine de soja ® | 5 g |
   | - NaHCO3 | 0,35 g |
   | - L-Glutamine | 0,75 g |
   | - HEPES | 5,95 g |
   | - D(+) glucose | 2,50 g |
   | - H20 | Q.S. 800 ml |
   | - Milieu 199 H modifié® (x10) | 4 ml (5%) |
   | - Hémine porcine irradié à 25 kilogray | 0,003 mM |
   | - Glutathion réduit | 0,08 mM |
   | - Solution de vitamines (x100) | 2 % |

   pour les promastigotes :

   | | |
   |---|---|
   | RPMI 1640 (1x) | 1000 ml |
   | Milieu 199 H modifié 10 x | 2 % |
   | Hémine porcine irradiée à 25 kilogray : | 0,0002% |
   | Gentamicine sulfate | 0.04 mg |

   et d'autre part du muramyl dipeptide

2. Complexe vaccinal thérapeutique destiné à la prévention ou au traitement des leishmanioses et des infections à microorganismes pathogènes intracellulaires chez le mammifère, selon la revendication 1, **caractérisé en ce que** le muramyl dipeptide est associé aux molécules d'excrétion sécrétion issues de promastigotes et/ou d'amastigotes de Leishmania sp. dans un rapport protéine/adjuvant de 1/0,5 à 1/4.

3. Complexe vaccinal thérapeutique destiné à la prévention ou au traitement des leishmanioses et des infections à microorganismes pathogènes intracellulaires chez les canidés, selon la revendication 1 ou 2, **caractérisé en ce que** le muramyl dipeptide est associé aux molécules d'excrétion sécrétion issues de promastigotes et/ou d'amastigotes de Leishmania sp. dans un rapport de 100 $\mu$g de protéines pour 200 $\mu$g de muramyl dipeptide.

4. Complexe vaccinal thérapeutique destiné à la prévention ou au traitement des leishmanioses et des infections à microorganismes pathogènes intracellulaires chez les mammifères, selon l'une quelconque des revendications 1 à 3 **caractérisé en ce que** les molécules d'excrétion sécrétion sont obtenues par un procédé au cours duquel est réalisée une filtration tangentielle contre une membrane de 0,16$\mu$ en polyethersulfone pour la séparation des parasites puis une concentration par un facteur 100 du filtrat par filtration tangentielle contre un filtre de 3 kDa en polyethersulfone

5. Complexe vaccinal thérapeutique destiné à la prévention ou au traitement des leishmanioses et des infections à microorganismes pathogènes intracellulaires chez le mammifère, selon l'une quelconque des revendications 1 à 4, ayant la particularité d'induire chez le mammifère une activité leishmanicide des monocytes parasités mesurables selon la méthode de LEMESRE de détermination de l'inhibition de l'index parasitaire à 48 heures d'incubation.

**6.** Complexe vaccinal thérapeutique destiné à la prévention ou au traitement des leishmanioses et des infections à microorganismes pathogènes intracellulaires chez le mammifère, selon l'une quelconque des revendications 1 à 5, ayant la particularité d'induire chez le mammifère une activation de l'immunité à médiation cellulaire dépendante de lymphocytes T et préferentiellement de lymphocytes T de type Th1.

**7.** Complexe vaccinal thérapeutique destiné à la prévention ou au traitement des leishmanioses et des infections à microorganismes pathogènes intracellulaires chez le mammifère selon l'une quelconque des revendications 1 à 6, ayant la particularité d'induire chez le mammifère le passage d'un état immunitaire de type Th2 vers un état immunitaire de type Th1.

**8.** Complexe vaccinal thérapeutique destiné à la prévention ou au traitement des leishmanioses et des infections à microorganismes pathogènes intracellulaires chez le mammifère, selon l'une des revendications 6 ou 7, ayant la particularité d'induire chez le mammifère des anticorps spécifiques et plus particulièrement des anticorps spécifiques d'isotype IgG2.

**9.** Complexe vaccinal thérapeutique destiné à la prévention ou au traitement des leishmanioses et des infections à microorganismes pathogènes intracellulaires chez le mammifère, selon l'une quelconque des revendications 1 à 8, **caractérisé en ce que** les molécules d'excrétion sécrétion issues de promastigotes et/ou d'amastigotes de Leishmania sp, présentent au moins un épitope porté par la protéine immunogène majeure chez le chien

**10.** Complexe vaccinal thérapeutique destiné à la prévention ou au traitement des leishmanioses et des infections à microorganismes pathogènes intracellulaires chez les mammifères, selon l'une quelconque des revendications 1 à 9, **caractérisé en ce que** certaines des molécules d'excrétion sécrétion issues de promastigotes et/ou amastigotes de Leishmania sp. présentent des activités protéasiques non répertoriées (ni métallo, ni sérine, ni cystéine protéase ).

**11.** Complexe vaccinal thérapeutique destiné à la prévention ou au traitement des leishmanioses et des infections à microorganismes pathogènes intracellulaires chez le mammifère, selon l'une quelconque des revendications 1 à 10, conditionné sous une forme telle qu'il peut être administré par différentes voies : sous cutanée, intradermique, intramusculaire, intraveineuse, parentale et orale.

**Patentansprüche**

**1.** Therapeutischer Impfstoftkomplex für die Prävention oder die Behandlung von Leishmaniosen und durch pathogene intrazelluläre Mikroorganismen hervorgerufene Infektionen beim Säuger, insbesondere bei Menschen, Kaniden, Feliden und Equiden, **dadurch gekennzeichnet, dass** er zusammengesetzt ist aus:

- einerseits Exkretions-Sekretions-Molekülen aus Amastigoten und/oder Promastigoten von Leishmania sp. , die in axenischem und serumfreiem Medium folgender, vollständig beschriebener Zusammensetzung hergestellt werden:

für die Amastigoten (Mengen pro 800 ml) :

| | |
|---|---|
| - Medium 199 H ® (x10) ( mit Hanks' Salzen) | 100 ml |
| - Soja-Tryptokasein ® | 5 g |
| - NaHCO$_3$ | 0,35 g |
| - L-Glutamin | 0,75 g |
| - HEPES | 5,95 g |
| - D(+) Glucose | 2,50 g |
| - H20 | q.s. 800 ml |
| - Modifiziertes Medium 199 H ® (x10) | 4 ml (5%) |
| - mit 25 Kilogray bestrahltes Schweinehämin | 0,003 mM |
| - reduziertes Glutathion | 0,08 mM |
| - Vitaminlösung (x100) | 2 % |

für die Promastigoten :

| | |
|---|---|
| RPMI1640 (1x) | 1000 ml |
| Modifiziertes Medium 199 H 10 x | 2% |
| mit 25 Kilogray bestrahltes Schweinehämin | 0,0002% |
| Gentamycinsulfat | 0,04 mg |

und andererseits aus Muramyldipeptid.

2.  Therapeutischer Impfstoflkomplex für die Prävention oder die Behandlung von Leishmaniosen und durch pathogene intrazelluläre Mikroorganismen hervorgerufene Infektionen beim Säuger nach Anspruch 1, **dadurch gekennzeichnet, dass** das Muramyldipeptid den Exkretions-Sekretions-Molekülen aus Promastigoten und/oder Amastigoten von Leishmania sp. in einem Verhältnis Protein-Adjuvans von 1:0,5 bis 1:4 zugegeben wird.

3.  Therapeutischer Impfstoffkomplex für die Prävention oder die Behandlung von Leishmaniosen und durch pathogene intrazelluläre Mikroorganismen hervorgerufene Infektionen bei Kaniden nach Anspruch 1 oder 2, **dadurch gekennzeichnet, dass** das Muramyldipeptid den Exkretions-Sekretions-Molekülen aus Promastigoten und/oder Amastigoten von Leishmania sp. in einem Verhältnis von 100 $\mu$g Protein pro 200 $\mu$g Muramyldipeptid zugegeben wird.

4.  Therapeutischer Impfstoflkomplex für die Prävention oder die Behandlung von Leishmaniosen und durch pathogene intrazelluläre Mikroorganismen hervorgerufene Infektionen beim Säuger nach einem der Ansprüche 1 bis 3, **dadurch gekennzeichnet, dass** die Exkretions-Sekretions-Moleküle mit Hilfe eines Verfahrens gewonnen werden, bei dem zum Zweck der Separation der Parasiten eine tangentiale Filtration mit einer 0,16-$\mu$-Polyethersulfonmembran und danach mittels tangentialer Filtration mit einem 3 kDa-Filter aus Polyethersulfon eine Konzentration des Filtrats um einen Faktor 100 erfolgt.

5.  Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 4, mit der Besonderheit, dass beim Säuger eine leishmanizide Wirkung der nach 48-stündiger Inkubation nach der Methode von LEMESRE zur Bestimmung der Inhibition des Parasitenindex messbaren parasiteninduzierten Monozyten herbeigeführt wird.

6.  Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 5, mit der Besonderheit, dass beim Säuger eine zellulär vermittelte, von T-Lymphozyten, vorzugsweise von T-Lymphozyten vom Typ Th1 abhängige Immunaktivierung herbeigeführt wird.

7.  Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 6, mit der Besonderheit, dass beim Säuger der Übergang von einem Immunstatus vom Typ Th2 zu einem Immunstatus vom Typ Th1 herbeigeführt wird.

8.  Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 7, mit der Besonderheit, dass beim Säuger spezifische Antikörper, im besonderen spezifische Antikörper vom Isotyp IgG2 induziert werden.

9.  Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 8, **dadurch gekennzeichnet, dass** die Exkretions-Sekretions-Moleküle aus Promastigoten und/oder Amastigoten von Leishmania sp. beim Hund mindestens ein Epitop auf dem Hauptoberflächenprotein aufweisen.

10. Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 9, **gekennzeichnet dadurch, dass** einige Exkretions-Sekretions-Moleküle aus Promastigoten und/oder Amastigoten von Leishmania sp. nicht registrierte Proteinaseaktivitäten (weder Metallo- noch Serin- noch Cysteinproteinase) aufweisen.

**11.** Therapeutischer Impfstoffkomplex für die Prävention oder die Behandlung von Leishmaniosen und durch pathogene intrazelluläre Mikroorganismen hervorgerufene Infektionen beim Säuger nach einem der Ansprüche 1 bis 10, der in der Weise aufbereitet ist, dass er auf verschiedenen Wegen verabreicht werden kann: subkutan, interdermal, intramuskulär, intravenös, parenteral und oral.

**Claims**

**1.** A therapeutic vaccine complex intended for the prevention or treatment of leishmaniases and infections with intracellular pathogenic micro-organisms in mammals and in particular humans, canidae, felidae and equidae, **characterised in that** it is composed of

- on the one hand excretion secretion molecules from amastigotes and/or promastigotes of Leishmania sp. produced in an axenic and aseric medium which is perfectly defined such that the medium comprises:

for the amastigotes (amounts for 800 ml):

| - medium 199 H® (x10) (with Hanks salts) | 100 ml |
|---|---|
| - soya tripto-casein® | 5 g |
| - NaHCO3 | 0.35 g |
| - L-glutamine | 0.75 g |
| - HEPES | 5.95 g |
| - D(+) glucose | 2.50 g |
| - H2O | Q.S. 800 ml |
| - modified medium 199 H® (x10) | 4 ml (5%) |
| - porcine haemine irradiated at 25 kilogray | 0.003 mM |
| - reduced glutathione | 0.08 mM |
| - solution of vitamins (x100) | 2% |

for the promastigotes:

| RPMI 1640 (1x) | 1000 ml |
|---|---|
| modified medium 199 H 10 x | 2% |
| porcine haemine irradiated at 25 kilogray: | 0.0002% |
| gentamicin sulphate | 0.04 mg |

and on the other hand muramyl dipeptide.

**2.** A therapeutic vaccine complex intended for the prevention or treatment of leishmaniases and infections with intracellular pathogenic micro-organisms in mammals according to claim 1 **characterised in that** the muramyl dipeptide is associated with the excretion secretion molecules from promastigotes and/or amastigotes of Leishmania sp. in a protein/adjuvant ratio of 1/0.5 to 1/4.

**3.** A therapeutic vaccine complex intended for the prevention or treatment of leishmaniases and infections with intracellular pathogenic micro-organisms in canidae according to claim 1 or claim 2 **characterised in that** the muramyl dipeptide is associated with the excretion secretion molecules from promastigotes and/or amastigotes of Leishmania sp. in a ratio of 100 $\mu$g of proteins for 200 $\mu$g of muramyl dipeptide.

**4.** A therapeutic vaccine compiex intended for the prevention or treatment of leishmaniases and infections with intracellular pathogenic micro-organisms in mammals according to any one of claims 1 to 3 **characterised in that** the excretion secretion molecules are obtained by a process, in the course of which tangential filtration is effected against a 0.16 μ membrane of polyethersulphone for the separation of parasites and then concentration by a factor of 100 of the filtrate by tangential filtration against a 3 kDa filter of polyethersulphone.

5. A therapeutic vaccine complex intended for the prevention or treatment of leishmaniases and infections with intracellular pathogenic micro-organisms in mammals according to any one of claims 1 to 4 having the particularity of inducing in mammals a leishmanicidal activity in respect of the parasitised monocytes which can be measured in accordance with the LEMESRE method for determining inhibition of the parasitic index at 48 hours of incubation.

6. A therapeutic vaccine complex intended for the prevention or treatment of leishmaniases and infections with intracellular pathogenic micro-organisms in mammals according to any one of claims 1 to 5 having the particularity of inducing in mammals activation of immunity to dependent cellular mediation of T lymphocytes and preferably T lymphocytes of type Th1.

7. A therapeutic vaccine complex intended for the prevention or treatment of leishmaniases and infections with intracellular pathogenic micro-organisms in mammals according to any one of claims 1 to 6 having the particularity of inducing in mammals the passage from an immunitary state of type Th2 towards an immunitary state of type Th1.

8. A therapeutic vaccine complex intended for the prevention or treatment of leishmaniases and infections with intracellular pathogenic micro-organisms in mammals according to one of claims 6 and 7 having the particularity of inducing in mammals specific antibodies and more particularly specific antibodies of isotype IgG2.

9. A therapeutic vaccine complex intended for the prevention or treatment of leishmaniases and infections with intracellular pathogenic micro-organisms in mammals according to any one of claims 1 to 8 **characterised in that** the excretion secretion molecules from promastigotes and/or amastigotes of Leishmania sp. have at least one epitope carried by the major immunogenic protein in dogs.

10. A therapeutic vaccine complex intended for the prevention or treatment of leishmaniases and infections with intracellular pathogenic micro-organisms in mammals according to any one of claims 1 to 9 **characterised in that** some of the excretion secretion molecules from promastigotes and/or amastigotes of Leishmania sp. have non-repertoried proteasic activities (neither metallo, nor serine nor cysteine protease).

11. A therapeutic vaccine complex intended for the prevention or treatment of leishmaniases and infections with intracellular pathogenic micro-organisms in mammals according to any one of claims 1 to 10 packaged in a form such that it can be administered in different ways: subcutaneous, intradermic, intramuscular, intravenous, parenteral or oral.

**Comparaison des milieux mA1 et MA1m pour la culture des amastigotes**

fig. 1

**Comparaison des milieux MP et MPm pour la culture des promastigotes**

fig. 2

fig. 3

fig. 4a

fig. 4b

fig. 5

fig. 6

**Etude effet dose : Réponse en IgG2**
( la moyenne des DO est effectuées pour chaque groupe de chiens ).

fig. 7